# EUROPÄISCHE PATENTANMELDUNG

(11) **EP 2 450 382 A1**
(43) Veröffentlichungstag der Anmeldung: **09.05.2012**
(21) Anmeldenummer: 10189967.2
(22) Anmeldetag: 04.11.2010
(51) Int. Cl.: C07K 16/40, C12N 9/64, A61K 47/48

(54) **Immunogenes Peptid**

(71) Anmelder: Affiris AG, 1030 Wien (AT)
(72) Erfinder: Brunner, Sylvia, 1080 Wien (AT); Galabova, Gergana, 1090 Wien (AT); Schmidt, Walter, 1020 Wien (AT); Wanko, Bettina, 1040 Wien (AT); Winsauer, Gabriele, 1050 Wien (AT)
(74) Vertreter: Sonn & Partner Patentanwälte

(57) **Zusammenfassung**

Die vorliegende Erfindung betrifft ein Peptid, welches aus 10 bis 30 Aminosäureresten besteht und von der Aminosäuresequenz SIPWNLERITPPRYRADEYQPPDGGSLVEV (SEQ ID Nr. 1) abgeleitet ist, wobei das Peptid die Aminosäuresequenz SIPWNLERIT umfasst, und ein Vakzin umfassend dieses Peptid.

SEQUENCE LISTING

<110>  Affiris AG

<120>  Immunogenes Peptid

<130>  R 57512

<160>  43

<170>  PatentIn version 3.5

<210>  1
<211>  30
<212>  PRT
<213>  Artificial Sequence

<220>
<223>  Fragment of PCSK9

<400>  1

Ser Ile Pro Trp Asn Leu Glu Arg Ile Thr Pro Pro Arg Tyr Arg Ala
1               5                   10                  15

Asp Glu Tyr Gln Pro Pro Asp Gly Gly Ser Leu Val Glu Val
            20                  25                  30

<210>  2
<211>  13
<212>  PRT
<213>  Artificial Sequence

<220>
<223>  Fragment of PCSK9

<400>  2

Ser Ile Pro Trp Asn Leu Glu Arg Ile Thr Pro Pro Arg
1               5                   10

<210>  3
<211>  12
<212>  PRT
<213>  Artificial Sequence

<220>
<223>  Fragment of PCSK9

<400>  3

Ile Pro Trp Asn Leu Glu Arg Ile Thr Pro Pro Arg
1               5                   10

<210>  4
<211>  11
<212>  PRT
<213>  Artificial Sequence

<220>
<223>  Fragment of PCSK9

<400>  4

Pro Trp Asn Leu Glu Arg Ile Thr Pro Pro Arg
1               5                   10

<210>  5
<211>  10
<212>  PRT
<213>  Artificial Sequence

<220>
<223>  Fragment of PCSK9

<400>  5

Trp Asn Leu Glu Arg Ile Thr Pro Pro Arg
1               5                   10


<210>  6
<211>  9
<212>  PRT
<213>  Artificial Sequence

<220>
<223>  Fragment of PCSK9

<400>  6

Asn Leu Glu Arg Ile Thr Pro Pro Arg
1               5


<210>  7
<211>  12
<212>  PRT
<213>  Artificial Sequence

<220>
<223>  Fragment of PCSK9

<400>  7

Ser Ile Pro Trp Asn Leu Glu Arg Ile Thr Pro Pro
1               5                   10


<210>  8
<211>  11
<212>  PRT
<213>  Artificial Sequence

<220>
<223>  Fragment of PCSK9

<400>  8

Ser Ile Pro Trp Asn Leu Glu Arg Ile Thr Pro
1               5                   10


<210>  9
<211>  10
<212>  PRT
<213>  Artificial Sequence

<220>
<223>  Fragment of PCSK9

<400>  9

Ser Ile Pro Trp Asn Leu Glu Arg Ile Thr
1               5                   10

<210>  10
<211>  9
<212>  PRT
<213>  Artificial Sequence

<220>
<223>  Fragment of PCSK9

<400>  10

Ser Ile Pro Trp Asn Leu Glu Arg Ile
1               5


<210>  11
<211>  11
<212>  PRT
<213>  Artificial Sequence

<220>
<223>  Fragment of PCSK9

<400>  11

Ile Pro Trp Asn Leu Glu Arg Ile Thr Pro Pro
1               5                   10


<210>  12
<211>  9
<212>  PRT
<213>  Artificial Sequence

<220>
<223>  Fragment of PCSK9

<400>  12

Pro Trp Asn Leu Glu Arg Ile Thr Pro
1               5


<210>  13
<211>  29
<212>  PRT
<213>  Artificial Sequence

<220>
<223>  Fragment of PCSK9

<400>  13

Ser Ile Pro Trp Asn Leu Glu Arg Ile Thr Pro Pro Arg Tyr Arg Ala
1               5                   10                  15


Asp Glu Tyr Gln Pro Pro Asp Gly Gly Ser Leu Val Glu
            20                  25


<210>  14
<211>  28
<212>  PRT
<213>  Artificial Sequence

<220>
<223>  Fragment of PCSK9

<400>  14

Ser Ile Pro Trp Asn Leu Glu Arg Ile Thr Pro Pro Arg Tyr Arg Ala
1               5                   10                  15

Asp Glu Tyr Gln Pro Pro Asp Gly Gly Ser Leu Val
            20                  25

<210>  15
<211>  27
<212>  PRT
<213>  Artificial Sequence

<220>
<223>  Fragment of PCSK9

<400>  15

Ser Ile Pro Trp Asn Leu Glu Arg Ile Thr Pro Pro Arg Tyr Arg Ala
1               5                   10                  15

Asp Glu Tyr Gln Pro Pro Asp Gly Gly Ser Leu
            20                  25

<210>  16
<211>  26
<212>  PRT
<213>  Artificial Sequence

<220>
<223>  Fragment of PCSK9

<400>  16

Ser Ile Pro Trp Asn Leu Glu Arg Ile Thr Pro Pro Arg Tyr Arg Ala
1               5                   10                  15

Asp Glu Tyr Gln Pro Pro Asp Gly Gly Ser
            20                  25

<210>  17
<211>  25
<212>  PRT
<213>  Artificial Sequence

<220>
<223>  Fragment of PCSK9

<400>  17

Ser Ile Pro Trp Asn Leu Glu Arg Ile Thr Pro Pro Arg Tyr Arg Ala
1               5                   10                  15

Asp Glu Tyr Gln Pro Pro Asp Gly Gly
            20                  25

<210>  18
<211>  24
<212>  PRT
<213>  Artificial Sequence

<220>

<223>  Fragment of PCSK9

<400>  18

Ser Ile Pro Trp Asn Leu Glu Arg Ile Thr Pro Pro Arg Tyr Arg Ala
1               5                   10                  15

Asp Glu Tyr Gln Pro Pro Asp Gly
            20

<210>  19
<211>  23
<212>  PRT
<213>  Artificial Sequence

<220>
<223>  Fragment of PCSK9

<400>  19

Ser Ile Pro Trp Asn Leu Glu Arg Ile Thr Pro Pro Arg Tyr Arg Ala
1               5                   10                  15

Asp Glu Tyr Gln Pro Pro Asp
            20

<210>  20
<211>  22
<212>  PRT
<213>  Artificial Sequence

<220>
<223>  Fragment of PCSK9

<400>  20

Ser Ile Pro Trp Asn Leu Glu Arg Ile Thr Pro Pro Arg Tyr Arg Ala
1               5                   10                  15

Asp Glu Tyr Gln Pro Pro
            20

<210>  21
<211>  21
<212>  PRT
<213>  Artificial Sequence

<220>
<223>  Fragment of PCSK9

<400>  21

Ser Ile Pro Trp Asn Leu Glu Arg Ile Thr Pro Pro Arg Tyr Arg Ala
1               5                   10                  15

Asp Glu Tyr Gln Pro
            20

<210>  22
<211>  20
<212>  PRT
<213>  Artificial Sequence

<220>
<223>  Fragment of PCSK9

<400>  22

Ser Ile Pro Trp Asn Leu Glu Arg Ile Thr Pro Pro Arg Tyr Arg Ala
1               5                   10                  15

Asp Glu Tyr Gln
            20

<210>  23
<211>  19
<212>  PRT
<213>  Artificial Sequence

<220>
<223>  Fragment of PCSK9

<400>  23

Ser Ile Pro Trp Asn Leu Glu Arg Ile Thr Pro Pro Arg Tyr Arg Ala
1               5                   10                  15

Asp Glu Tyr

<210>  24
<211>  18
<212>  PRT
<213>  Artificial Sequence

<220>
<223>  Fragment of PCSK9

<400>  24

Ser Ile Pro Trp Asn Leu Glu Arg Ile Thr Pro Pro Arg Tyr Arg Ala
1               5                   10                  15

Asp Glu

<210>  25
<211>  17
<212>  PRT
<213>  Artificial Sequence

<220>
<223>  Fragment of PCSK9

<400>  25

Ser Ile Pro Trp Asn Leu Glu Arg Ile Thr Pro Pro Arg Tyr Arg Ala
1               5                   10                  15

Asp

<210>  26
<211>  16

<212>  PRT
<213>  Artificial Sequence

<220>
<223>  Fragment of PCSK9

<400>  26

Ser Ile Pro Trp Asn Leu Glu Arg Ile Thr Pro Pro Arg Tyr Arg Ala
1               5                   10                  15


<210>  27
<211>  15
<212>  PRT
<213>  Artificial Sequence

<220>
<223>  Fragment of PCSK9

<400>  27

Ser Ile Pro Trp Asn Leu Glu Arg Ile Thr Pro Pro Arg Tyr Arg
1               5                   10                  15


<210>  28
<211>  14
<212>  PRT
<213>  Artificial Sequence

<220>
<223>  Fragment of PCSK9

<400>  28

Ser Ile Pro Trp Asn Leu Glu Arg Ile Thr Pro Pro Arg Tyr
1               5                   10


<210>  29
<211>  12
<212>  PRT
<213>  Artificial Sequence

<220>
<223>  Fragment of PCSK9

<400>  29

Tyr Arg Ala Asp Glu Tyr Gln Pro Pro Asp Gly Gly
1               5                   10


<210>  30
<211>  16
<212>  PRT
<213>  Artificial Sequence

<220>
<223>  Fragment of PCSK9

<400>  30

Thr Ser Ile Gln Ser Asp His Arg Glu Ile Glu Gly Arg Val Met Val
1               5                   10                  15


<210>  31
<211>  14

<212>  PRT
<213>  Artificial Sequence

<220>
<223>  Fragment of PCSK9

<400>  31

Glu Asn Val Pro Glu Glu Asp Gly Thr Arg Phe His Arg Gln
1               5                   10


<210>  32
<211>  16
<212>  PRT
<213>  Artificial Sequence

<220>
<223>  Fragment of PCSK9

<400>  32

Ala Gly Val Val Ser Gly Arg Asp Ala Gly Val Ala Lys Gly Ala Ser
1               5                   10                  15


<210>  33
<211>  7
<212>  PRT
<213>  Artificial Sequence

<220>
<223>  Fragment of PCSK9

<400>  33

Val Gln Pro Val Gly Pro Leu
1               5


<210>  34
<211>  14
<212>  PRT
<213>  Artificial Sequence

<220>
<223>  Fragment of PCSK9

<400>  34

Val Gly Ala Thr Asn Ala Gln Asp Gln Pro Val Thr Leu Gly
1               5                   10


<210>  35
<211>  16
<212>  PRT
<213>  Artificial Sequence

<220>
<223>  Fragment of PCSK9

<400>  35

Ile Ile Gly Ala Ser Ser Asp Cys Ser Thr Cys Phe Val Ser Gln Ser
1               5                   10                  15


<210>  36
<211>  14

<212>  PRT
<213>  Artificial Sequence

<220>
<223>  Fragment of PCSK9

<400>  36

Glu Ala Trp Phe Pro Glu Asp Gln Arg Val Leu Thr Pro Asn
1               5                   10


<210>  37
<211>  16
<212>  PRT
<213>  Artificial Sequence

<220>
<223>  Fragment of PCSK9

<400>  37

Ala Leu Pro Pro Ser Thr His Gly Ala Gly Trp Gln Leu Phe Cys Arg
1               5                   10                  15


<210>  38
<211>  18
<212>  PRT
<213>  Artificial Sequence

<220>
<223>  Fragment of PCSK9

<400>  38

Thr Val Trp Ser Ala His Ser Gly Pro Thr Arg Met Ala Thr Ala Ile
1               5                   10                  15


Ala Arg



<210>  39
<211>  15
<212>  PRT
<213>  Artificial Sequence

<220>
<223>  Fragment of PCSK9

<400>  39

Cys Ser Ser Phe Ser Arg Ser Gly Lys Arg Arg Gly Glu Arg Met
1               5                   10                  15


<210>  40
<211>  17
<212>  PRT
<213>  Artificial Sequence

<220>
<223>  Fragment of PCSK9

<400>  40

His Val Leu Thr Gly Cys Ser Ser His Trp Glu Val Glu Asp Leu Gly
1               5                   10                  15

Thr

<210>  41
<211>  14
<212>  PRT
<213>  Artificial Sequence

<220>
<223>  Fragment of PCSK9

<400>  41

Pro Val Leu Arg Pro Arg Gly Gln Pro Asn Gln Cys Val Gly
1               5                   10

<210>  42
<211>  10
<212>  PRT
<213>  Artificial Sequence

<220>
<223>  Fragment of PCSK9

<400>  42

Ser Ala Leu Pro Gly Thr Ser His Val Leu
1               5                   10

<210>  43
<211>  19
<212>  PRT
<213>  Artificial Sequence

<220>
<223>  Fragment of PCSK9

<400>  43

Arg Asp Val Ser Thr Thr Gly Ser Thr Ser Glu Glu Ala Val Thr Ala
1               5                   10                  15

Val Ala Ile

## Beschreibung

Die vorliegende Erfindung betrifft ein Vakzin zur Behandlung oder Verhinderung von gesundheitlichen Problemen, die durch Atherosklerose verursacht werden können, insbesondere Schlaganfall, kardiovaskuläre Erkrankungen (die zu Herzinfarkt führen können), sowie Erkrankungen peripherer Gefäße ("peripheral vascular disease").

Erkrankungen aufgrund von Atherosklerose, vor allem Erkrankungen der Herzkranzgefäße, sind mit ∼50% die häufigste Todesursache in der westlichen Welt. Atherosklerose ist unter anderem durch die Ablagerung von Lipid-Partikeln in großen Arterien gekennzeichnet. In einem vielstufigen Prozess, der über viele Jahre bis Jahrzehnte andauert, führt Atherosklerose zur Verengung der betroffenen Gefäße. Je nach Lage der Gefäße können kardiovaskuläre Erkrankungen (Herzinfarkt), Schlaganfall und periphere vaskuläre Erkrankungen die Folge sein. Eine Hauptursache für Atherosklerose sind durch Lebensstil (v.a. wenig Bewegung, fettreiche Ernährung, Rauchen) und/oder genetische Faktoren bedingte Probleme mit dem Fettstoffwechsel, d.h. zu hohes Gesamt-Cholesterin beziehungsweise insbesondere zu hohe Werte an LDLc ("Low Density Lipoprotein Cholesterol") in Kombination mit niedrigen HDLc-Werten ("High Density Lipoprotein Cholesterol").

Hohe LDLc-Werte korrelieren direkt mit dem Auftreten von kardiovaskulären Erkrankungen. Die häufigste Form von erblich bedingten zu hohen LDLc-Spiegeln ist die Autosomal-dominante Hypercholesterinämie (ADH). Damit in Zusammenhang stehen drei Gene: der LDL-Rezeptor (LDLR), Apolipoprotein B-100 (der Ligand für den LDLR) sowie die Proteinkonvertase PCSK9 ("proprotein convertase subtilisin kexin 9").

Zur Familie der sekretorischen Proproteinkonvertasen (PC) gehören sieben für basische Aminosäuren spezifische Subtilisin-ähnliche Serin-Proteinasen ("subtilisin-like serine proteinases"), nämlich PC1/3, PC2, Furin, PC4, PC5/6, PACE4 and PC7. Au-βerdem gehören zwei weitere PC dazu, die an nicht-basischen Aminosäuren schneiden, nämlich SKI-1/S1P und PCSK9 (auch NARC-1 ("neural apoptosis-regulated convertase 1") genannt).

Mit Ausnahme von PC4 werden die Konvertasen im Gehirn und peripheren Organen exprimiert. Sie haben unterschiedliche Funktionen, beispielsweise für die Produktion von Neuropeptiden, Wachstumsfaktoren, Zytokinen, Rezeptoren, bei der Zelladhäsion und Zellmigration sowie Wachstum und Differenzierung von Vorläuferzellen. Für etliche Konvertasen ist bekannt, dass sie bei Erkrankungen wie Krebs, oder auch bei viralen Infektionen eine Rolle spielen. Konditionelles Ausschalten von PC5/6 in Mäusen führt zu Fehlbildungen und Knochendefekten in Embryos, was vermutlich auf ausbleibendes Prozessieren des Wachstumsdifferenzierungsfaktors 11 ("growth differention factor 11") zurückzuführen ist.

Einige Mitglieder der Familie der Konvertasen beeinflussen auch den Lipidstoffwechsel:
- SKI-1/S1P aktiviert die Synthese von Cholesterin und Fettsäuren sowie des LDL Rezeptors (LDLR)
- Furin, PC5 und PACE4 inaktivieren die endotheliale Lipase sowie die Lipoprotein-Lipase
- PCSK9 vermittelt den Abbau des Low Density Lipoprotein Rezeptors (LDLR).

Das Gen für das humane PCSK9-Protein ist auf Chromosom 1p32.3 lokalisiert. Das Gen ist circa 22 Kilo-Basenpaare lang und kodiert für ein 692 Aminosäuren (AS) langes Glykoprotein. Die Expression von PCSK9 wird durch "sterol regulatory element binding proteins" (SREBPs) und Statine reguliert, was auch bei anderen im Cholesterinstoffwechsel wichtigen Genen der Fall ist. PCSK9 wird hauptsächlich in der Leber, aber auch in Dünndarm und Niere exprimiert und sekretiert. Das 74 kDa große Proprotein wird im endoplasmatischen Retikulum autokatalytisch prozessiert, dabei entsteht das etwa 60 kDa große Protein.

PCSK9 besteht aus
- einem 30 AS langen Signalpeptid
- der Propeptid- oder inhibitorischen Domäne (AS 31-152)
- der "subtilisin-like catalytic domain" (AS 153-452) und
- der C-terminalen Domäne ("cystein-rich unique C-terminal domain", CRD, AS 453-692).

PCSK9 spielt eine entscheidende Rolle im Cholesterinstoffwechsel, da es direkt die Menge an vorhandenem LDLR auf den Leberzellen reguliert. Der LDLR ist ein in der Plasmamembran lokalisiertes Glykoprotein, das cholesterinreiche LDLc-Partikel durch Endozytose aus dem Plasma entfernt. Das Binden von PCSK9 führt zur Aufnahme des LDLR in die Leberzellen und anschließend zu dessen Abbau in Lysosomen, im Gegensatz zum Recycling Richtung Zelloberfläche, das ohne Bindung von PCSK9 stattfinden würde.

Zusätzlich scheint PCSK9 einen Einfluss auf die Rezeptoren für ApoE (ApoER2) und VLDL ("Very Low Density Lipoprotein") zu haben. Dies konnte bisher allerdings nur *in vitro* gezeigt werden.

In *in vivo* Experimenten in Mäusen wurde gezeigt, dass die Menge an PCSK9 in humanem Plasma ausreicht, um die Menge an LDLR auf den Leberzellen zu reduzieren. Außerdem wurden in den letzten Jahren einige natürlich vorkommende Mutationen im PCSK9-Gen identifiziert, die zu verstärkter ("gain-of-function mutations") bzw. verminderter ("loss-of-function mutations") Aktivität des Proteins führen.

Sowohl im Menschen als auch in Mäusen führen "gain-of-function mutations" zu verringerten Mengen an LDLR in der Leber. Das verursacht große Mengen an LDLc im Plasma (Hypercholesterinämie), was eine Prädisposition für atherosklerotische Erkrankungen der Herzkranzgefäße (Koronare Herzkrankheit, KHK) hervorruft.

Die Über-Expression von PCSK9 in Mäusen führt zu höheren LDLc-Spiegeln. Auch die Administration von rekombinant hergestelltem PCSK9-Protein reduziert im Mausmodell die Anzahl der LDL-Rezeptoren dramatisch.

"Loss of function mutations" bewirken größere Mengen an LDLR, was zu geringeren LDLc Werten führt. Diese Hypocholesterinämie erweist sich als Schutz vor Erkrankungen der Herzkranzgefäße.

"Nonsense"-Mutationen sind in manchen Bevölkerungsgruppen häufig anzutreffen, z.B. haben circa 2% der Afro-Amerikaner eine von zwei Mutationen, die zu einer Verringerung der LDLc-Spiegel um ∼30% führt. In Kaukasiern ist eine "missense"-Mutation bekannt, die das LDLc um ∼15% erniedrigt. Diese geringeren LDLc Mengen führen zu einer deutlich verringerten Inzidenz an KHK.

PCSK9-"knock-out"-Mäuse haben mehr LDLR auf ihren Leberzellen und reduzierte Mengen an LDLc im Plasma. Auch eine Reduktion von PCSK9 mittels siRNA führt zu einer signifikanten Senkung von Gesamt-Cholesterin und LDLc in Mäusen und in einem Primatenmodell (Macaca fascicularis). Die selbe Wirkung, nämlich eine Reduktion von LDLc (in Mäusen und nicht-humanen Primaten) und Erhöhung von LDLR, konnte auch bei Anwendung von monoklonalen und polyklonalen Antikörpern, die die Bindung von PCSK9 an den LDLR verhindern, gezeigt werden.

Es sind derzeit keine negativen Auswirkungen eines Fehlens von PCSK9 bekannt. Angeborener homozygoter "knock-out" von PCSK9 führt im Menschen zu extrem geringen Werten an LDLc ohne erkennbare Nebenwirkungen. Die Reduktion von PCSK9, beispielsweise durch Antisense-Oligonukleotide, oder die Inaktivierung von PCSK9, beispielsweise durch "small molecule inhibitors" oder Antikörper, stellt daher ein attraktives Ziel dar. Damit sollen die Auswirkungen von Atherosklerose, vor allem kardiovaskuläre Erkrankungen, verringert werden. Dieser therapeutische Ansatz ist sowohl als Einzeltherapie als auch in Kombination z.B. mit Statinen (die das PCSK9-Protein hochregulieren) oder auch mit Medikamenten, die den HDLc Spiegel beeinflussen, vorstellbar.

Es ist Aufgabe der vorliegenden Erfindung, Mittel zur Verfügung zu stellen, die geeignet sind, die LDLR-Bindungsaktivität von PCSK9 und infolgedessen den Gehalt von LDLc zu reduzieren.

Die gegenständliche Erfindung betrifft ein Peptid, welches aus 10 bis 30 Aminosäureresten besteht und von der Aminosäuresequenz SIPWNLERITPPRYRADEYQPPDGGSLVEV (SEQ ID Nr. 1) abgeleitet ist, wobei das Peptid die Aminosäuresequenz SIPWNLERIT (SEQ ID Nr. 9) umfasst.

Es hat sich herausgestellt, dass ein Peptid mit der eingangs beschriebenen Aminosäuresequenz bei Verabreichung an einem Individuum in der Lage ist, die Bildung von Antikörpern gerichtet gegen PCSK9 zu induzieren, wodurch die Interaktion zwischen PCSK9 und LDL-Rezeptor signifikant reduziert wird, da die gebildeten Antikörper an PCSK9 binden. Dadurch wird der PCSK9-vermittelte Abbau von LDLR verhindert und die Menge an LDLc reduziert, was wiederum zur Folge hat, dass Erkrankungen, die Folge eines erhöhten LDLc-Levels sind (z.B. kardiovaskuläre Erkrankungen), behandelt bzw. vorbeugend behandelt werden können.

Das erfindungsgemäße Peptid ist von der Aminosäuresequenz SIPWNLERITPPRYRADEYQPPDGGSLVEV (SEQ ID Nr. 1) abgeleitet. "Abgeleitet" bedeutet in diesem Zusammenhang, dass das erfindungsgemäße Peptid ein Fragment eines Peptids mit der Aminosäuresequenz SIPWNLERITPPRYRADEYQPPDGGSLVEV (SEQ ID Nr. 1) ist.

Das erfindungsgemäße Peptid umfasst jedenfalls die ersten 10 Aminosäurereste von SEQ ID Nr. 1, da insbesondere dieser Bereich von PCSK9 in der Lage ist, bei Verabreichung an ein Individuum PCSK9 spezifische Antikörper zu induzieren, welche in der Lage sind, die Interaktion zwischen PCSK9 und LDL-Rezeptor spezifisch zu inhibieren. Es konnte experimentell festgestellt werden, dass ein Peptid, um die erfindungsgemäßen Eigenschaften zu entfalten, zumindest die Aminosäuresequenz SIPWNLERIT umfassen muss. Bereits das Weglassen des C-terminalen Threonins von SEQ ID Nr. 9 führt zu einem Peptid, welches in unzureichendem Maße in der Lage ist, die Bildung von Antikörpern zu induzieren, welche die erfindungsgemäße Aufgabe lösen.

Die erfindungsgemäßen Peptide können mit Verfahren hergestellt werden, die im Stand der Technik hinreichend beschrieben wurden. Die Peptide können sowohl rekombinant in entsprechenden Expressionssystemen als auch chemisch synthetisiert werden. Als geeignete Expressionssysteme eignen sich eukaryotische (z.B. Hefen, insbesondere Pichia pastoris) und prokaryotische Systeme (z.B. Bakterien, insbesondere E. coli), welche dem Fachmann hinreichend bekannt sind. Peptide lassen sich aber mit zahlreichen standardisierten Methoden auch chemisch synthetisieren, wobei für jedes einzelne Peptid eine individuelle Synthesestrategie ausgearbeitet werden muss. In der Regel werden die Peptide dabei an Festphasen synthetisiert, wobei die Arbeitsschritte Aktivierung einer N-terminal geschützten Aminosäure, Kopplung, Waschen, Entblockieren, Aktivierung usw. so oft durchlaufen werden, bis das gewünschte Peptid fertiggestellt ist. Dieses wird von der Festphase abgelöst, über Chromatographie wie z.B. HPLC (Hochleistungsflüssigkeitschromatographie) gereinigt und dann den weiteren Untersuchungen wie z.B. Verifizierung der Sequenz zugeführt.

Gemäß einer bevorzugten Ausführungsform der vorliegenden Erfindung weist das Peptid die Aminosäuresequenz SIPWNLERITPPRYRADEYQPPDGGSLVEV (SEQ ID Nr. 1), SIPWNLERITPPRYRADEYQPPDGGSLVE (SEQ ID Nr. 13), SIPWNLERITPPRYRADEYQPPDGGSLV (SEQ ID Nr. 14), SIPWNLERITPPRYRADEYQPPDGGSL (SEQ ID Nr. 15), SIPWNLERITPPRYRADEYQPPDGGS (SEQ ID Nr. 16), SIPWNLERITPPRYRADEYQPPDGG (SEQ ID Nr. 17), SIPWNLERITPPRYRADEYQPPDG (SEQ ID Nr. 18), SIPWNLERITPPRYRADEYQPPD (SEQ ID Nr. 19), SIPWNLERITPPRYRADEYQPP (SEQ ID Nr. 20), SIPWNLERITPPRYRADEYQP (SEQ ID Nr. 21), SIPWNLERITPPRYRADEYQ (SEQ ID Nr. 22), SIPWNLERITPPRYRADEY (SEQ ID Nr. 23), SIPWNLERITPPRYRADE (SEQ ID Nr. 24), SIPWNLERITPPRYRAD (SEQ ID Nr. 25), SIPWNLERITPPRYRA (SEQ ID Nr. 26), SIPWNLERITPPRYR (SEQ ID Nr. 27), SIPWNLERITPPRY (SEQ ID Nr. 28), SIPWNLERITPPR (SEQ ID Nr. 2), SIPWNLERITPP (SEQ ID Nr. 7), SIPWNLERITP (SEQ ID Nr. 8) oder SIPWNLERIT (SEQ ID Nr. 9) auf. In einer besonders bevorzugten Ausführungsform weist das Peptid die Aminosäuresequenz SIPWNLERITPPR (SEQ ID Nr. 2), SIPWNLERITPP (SEQ ID Nr. 7), SIPWNLERITP (SEQ ID Nr. 8) oder SIPWNLERIT (SEQ ID Nr. 9) auf.

Das erfindungsgemäße Peptid weist vorzugsweise am C-Terminus und/oder am N-Terminus einen Cysteinrest auf. Cysteinreste können beispielsweise zur Zyklisierung der Peptide verwendet werden. Ferner können an die SH-Gruppen der Cysteinreste, vorzugsweise an die N- oder C-terminalen Cysteinreste, weitere Substanzen gekoppelt werden. Der Cysteinrest kann dabei ein in natürlicher Weise an dieser Stelle vorkommender Cysteinrest sein oder aber am N- oder C-Terminus einer aus der nativen Sequenz entstammenden Sequenz angehängt sein. Es ist aber selbstverständlich auch möglich, über ein internes Cystein zu koppeln.

Das erfindungsgemäße Peptid kann gemäß einer bevorzugten Ausführungsform in einem Verfahren zur Behandlung und/oder Prävention von durch Atherosklerose verursachten Störungen bzw. Erkrankungen, insbesondere kardiovaskulären Erkrankungen, Schlaganfällen oder peripheren vaskulären Erkrankungen, verwendet werden.

Gemäß einer bevorzugten Ausführungsform der vorliegenden Erfindung wird das Peptid einem Patienten in einer Menge von 0,1 ng bis 10 mg, vorzugsweise von 1 µg bis 500 µg, verabreicht.

Ein weiterer Aspekt der vorliegenden Erfindung betrifft ein Vakzin umfassend ein Peptid gemäß der vorliegenden Erfindung.

Die erfindungsgemäßen Peptide eignen sich insbesondere zur Verwendung in einer Vakzinformulierung. Das erfindungsgemäße Vakzin kann nicht nur ein erfindungsgemäßes Peptid umfassen, sondern auch mindestens zwei, drei, vier, fünf, sechs oder zehn verschiedene Peptide ausgewählt aus der Gruppe bestehend aus SIPWNLERITPPRYRADEYQPPDGGSLVEV (SEQ ID Nr. 1), SIPWNLERITPPRYRADEYQPPDGGSLVE (SEQ ID Nr. 13), SIPWNLERITPPRYRADEYQPPDGGSLV (SEQ ID Nr. 14), SIPWNLERITPPRYRADEYQPPDGGSL (SEQ ID Nr. 15), SIPWNLERITPPRYRADEYQPPDGGS (SEQ ID Nr. 16), SIPWNLERITPPRYRADEYQPPDGG (SEQ ID Nr. 17), SIPWNLERITPPRYRADEYQPPDG (SEQ ID Nr. 18), SIPWNLERITPPRYRADEYQPPD (SEQ ID Nr. 19), SIPWNLERITPPRYRADEYQPP (SEQ ID Nr. 20), SIPWNLERITPPRYRADEYQP (SEQ ID Nr. 21), SIPWNLERITPPRYRADEYQ (SEQ ID Nr. 22), SIPWNLERITPPRYRADEY (SEQ ID Nr. 23), SIPWNLERITPPRYRADE (SEQ ID Nr. 24), SIPWNLERITPPRYRAD (SEQ ID Nr. 25), SIPWNLERITPPRYRA (SEQ ID Nr. 26), SIPWNLERITPPRYR (SEQ ID Nr. 27), SIPWNLERITPPRY (SEQ ID Nr. 28), SIPWNLERITPPR (SEQ ID Nr. 2), SIPWNLERITPP (SEQ ID Nr. 7), SIPWNLERITP (SEQ ID Nr. 8) und SIPWNLERIT (SEQ ID Nr. 9).

Das Peptid ist dabei vorzugsweise an einen pharmazeutisch akzeptablen Träger, vorzugsweise an KLH ("Keyhole Limpet Hemocyanin", Schlüsselloch-Napfschnecken-Hämocyanin), gekoppelt.

Weitere geeignete und vorzugsweise eingesetzte Träger sind Tetanus-Toxoid, Albumin-bindendes Protein, Serumalbumin, ein Dendrimer (MAP; Biol., Chem. 358: 581), Peptid-Linker (oder flankierende Regionen) sowie Adjuvans-Substanzen, die in Singh et al., Nat. Biotech. 17 (1999): 1075-1081 (insbesondere jene in Tabelle 1 dieses Dokuments) und O'Hagan et al., Nature Reviews Drug Discovery 2 (9) (2003): 727-735 geoffenbart sind (insbesondere die endogenen immunverstärkenden Verbindungen und Abgabesysteme, die darin beschrieben sind) oder Mischungen davon.

Außerdem kann das erfindungsgemäße Vakzin mit einem Adjuvans, vorzugsweise einer wenig löslichen Aluminium-Zusammensetzung, insbesondere Aluminiumhydroxid, formuliert werden. Natürlich können auch Adjuvantien wie MF59, Aluminiumphosphat, Calciumphosphat, Cytokine (z.B. IL-2, IL-12, GM-CSF), Saponine (z.B. QS21), MDP-Derivate, CpG-Oligos, LPS, MPL, Lipopep-tide'[Sylvia Br1], Polyphosphazene, Emulsionen (z.B. Freundsches Adjuvans, SAF), Liposome, Virosome, Iscome, Cochleate, PLG-Mikropartikel, Poloxamer-Partikel, Virus-artige Partikel, Hitzeinstabiles Enterotoxin (LT), Cholera-Toxin (CT), mutierte Toxine (z.B. LTK63 und LTR72), Mikropartikel und/oder polymerisierte Liposome, verwendet werden.

Weitere geeignete Adjuvantien können beispielsweise Purcell W et al. (Nature Reviews Drug Discovery 6 (2007): 404-414), insbesondere Box 2, entnommen werden.

Die Verbindung der vorliegenden Erfindung ist vorzugsweise an den Träger oder das Adjuvans über einen Linker gebunden, der ausgewählt ist aus der Gruppe der NHS-poly (Ethylenoxide) (PEO) (z.B. NHS-PEO4-Maleimid). Die Konjugations-Chemie (z.B. über heterobifunktionelle Verbindungen, wie GMBS, und natürlich auch andere, wie sie etwa in "Bioconjugate Techniques", Greg T. Hermanson beschrieben sind) kann in diesem Zusammenhang aus dem Fachmann bekannten Reaktionen ausgewählt werden. Die kovalente Konjugation wird vorzugsweise über den (die) N- oder C-terminale(n) Cysteinrest(e) etabliert.

Gemäß einer bevorzugten Ausführungsform ist das Peptid zur intradermalen, subkutanen oder intramuskulären Verabreichung formuliert.

Ein Vakzin, das das erfindungsgemäße Peptid umfasst, kann auf jede geeignete Anwendungsweise, beispielsweise i.d., i.p., i.m., intranasal, oral, subkutan, usw., und mit jeder geeigneten Abgabevorrichtung verabreicht werden (O'Hagan et al., Nature Reviews Drug Discovery 2 (9) (2003): 727-735). Das Peptid der vorliegenden Erfindung ist besonders bevorzugt zur subkutanen, intradermalen oder intramuskulären Verabreichung formuliert (vgl. z.B. "Handbook of Pharmaceutical Manufacturing Formulations", Sarfaraz Niazi, CRC Press Inc, 2004).

Typischerweise enthält das Vakzin das erfindungsgemäße Peptid in einer Menge von 0,1 ng bis 10 mg, vorzugsweise 10 ng bis 1 mg, insbesondere 100 ng bis 100 µg, oder alternativ z.B. 100 fmol bis 10 µmol, vorzugsweise 10 pmol bis 1 µmol, insbesondere 100 pmol bis 100 nmol. Typischerweise kann das Vakzin auch Hilfssubstanzen, z.B. Puffer, Stabilisatoren usw., enthalten.

Das erfindungsgemäße Vakzin kann in einem Verfahren zur Behandlung und/oder Prävention von durch Atherosklerose verursachten Störungen, insbesondere kardiovaskulären Erkrankungen, Schlaganfällen oder peripheren vaskulären Erkrankungen verwendet werden.

Das erfindungsgemäße Vakzin kann neben den hierin beschriebenen Peptiden weitere antigene Moleküle umfassen, welche in der Lage sind, in einem Individuum die Produktion entsprechender Antikörper zu induzieren. Dabei ist es insbesondere bevorzugt, Peptide und Polypeptide zu verwenden, welche ebenfalls in der Lage sind, die Bildung von PCSK9 spezifischen Antikörpern zu induzieren. Entsprechende Peptide sind beispielsweise in der WO 2008/125623 geoffenbart. Das erfindungsgemäße Vakzin kann somit neben einem oder mehreren der erfindungsgemäßen Peptide zusätzlich mindestens ein Peptid (vorzugsweise mindestens zwei, mindestens drei, mindestens vier, mindestens fünf, Peptide) oder ein funktionelles Fragment davon, welches im Wesentlichen dieselben Eigenschaften im Hinblick auf die Bildung PCSK9 spezifischer Antikörper in einem Individuum aufweist wie das Peptid, ausgewählt aus der folgenden Tabelle umfassen[Sylvia Br2]:

| **SEQ ID Nr.** | **PCSK9 - Bereich** | **Aminosäuresequenz** |
|---|---|---|
| 29 | 166-177 | YRADEYQPPDGG |
| 30 | 187-202 | TSIQSDHREIEGRVMV |
| 31 | 206-219 | ENVPEEDGTRFHRQ |
| 32 | 231-246 | AGVVSGRDAGVAKGAS |
| 33 | 277-283 | VQPVGPL |
| 34 | 336-349 | VGATNAQDQPVTLG |
| 35 | 368-383 | IIGASSDCSTCFVSQS |
| 36 | 426-439 | EAWFPEDQRVLTPN |
| 37 | 443-458 | ALPPSTHGAGWQLFCR |
| 38 | 459-476 | TVWSAHSGPTRMATAIAR |
| 39 | 486-500 | CSSFSRSGKRRGERM |
| 40 | 557-573 | HVLTGCSSHWEVEDLGT |
| 41 | 577-590 | PVLRPRGQPNQCVG |
| 42 | 636-645 | SALPGTSHVL |
| 43 | 659-677 | RDVSTTGSTSEEAVTAVAI |

Das erfindungsgemäße Vakzin bzw. die erfindungsgemäßen Peptide können im Verfahren zur Reduzierung oder Verhinderung des Erkrankungsrisikos durch, aber nicht limitiert auf, kardiovaskuläre Erkrankungen, Schlaganfall und periphere vaskuläre Erkrankungen und Störungen im Lipidstoffwechsel im Menschen bzw. zur Erniedrigung von LDL Cholesterin, therapeutische oder prophylaktische Behandlung von Atherosklerose, Lipidstoffwechselstörungen und Ähnliches verwendet werden.

Die vorliegende Erfindung wird anhand der Zeichnungen und der folgenden Beispiele näher veranschaulicht, ohne jedoch auf diese beschränkt zu sein.
Fig. 1 zeigt einen PCSK9-LDLR Interaktions-Assay: Median (n=5) % der Inhibierung.
Fig. 2 zeigt einen PCSK9 Protein-ELISA: Median (n=5) der Antikörpertiter gegen humanes PCSK9.
Fig. 3 zeigt einen Peptid-ELISA: Median (n=5) der Antikörpertiter gegen das vakzinierte Peptid.

### BEISPIELE:

### Beispiel 1: Herstellung der Vakzine

Die von PCSK9 abgeleiteten Peptide wurden über GMBS (gamma-Maleimidobuttersäure-N-hydroxysuccinimidester) an KLH gekoppelt. 30 µg dieser Konjugate (die Mengenangabe bezieht sich aufs Peptid, d.h. es sind 30 µg Peptid) wurden mit Aluminiumhydroxid (Endkonzentration Alum ist 0,2%) gemischt. Der dabei verwendete Puffer war PBS ("phosphate buffered saline").

**Tabelle A: PCSK9-Peptide, die zur Herstellung der Vakzine verwendet wurden (sämtliche Peptide umfassten einen C-terminalen Cystein-Rest)**

| ***SEQ ID Nr.*** | ***Aminosäuresequenz*** |
|---|---|
| 2 | SIPWNLERITPPR |
| 3 | IPWNLERITPPR |
| 4 | PWNLERITPPR |
| 5 | WNLERITPPR |
| 6 | NLERITPPR |
| 7 | SIPWNLERITPP |
| 8 | SIPWNLERITP |
| 9 | SIPWNLERIT |
| 10 | SIPWNLERI |
| 11 | IPWNLERITPP |
| 12 | PWNLERITP |

### Beispiel 2: Immunisierung

Jeweils 5 Balb/c Mäuse wurden subkutan immunisiert. Anzahl der Injektionen 2 bis 4 mal. Intervall zwischen den Immunisierungen 2 Wochen. Injektionsvolumen 200 µl/Maus.

2 Wochen nach der 3. bzw nach der 4. Immunisierung wurde Blut abgenommen.

### Beispiel 3: ELISA ("Enzyme Linked Immunosorbent Assay")

Serum oder Plasma der immunisierten Mäuse wurde mittels ELISA auf Antikörper gegen das immunisierte Peptid und auf Antikörper gegen das PCSK9 Protein getestet.

### Peptid-ELISA:

Die Peptide wurden an BSA gekoppelt und in einer Konzentration von 1 µM auf ELISA Platten immobilisiert. Nach einem Abblocken der nicht besetzten Stellen wurden die immobilisierten Peptide mit Serum in verschiedenen Verdünnungen inkubiert. Die Detektion der gebundenen Antikörper erfolgte mit einem biotinylierten polyklonalen anti-Maus IgG Antikörper gefolgt von Streptavidin gekoppelt an HRP ("horseradish peroxidase"). Als Substrat für die Peroxidase wurde ABTS (2,2'-Azino-di-(3-ethylbenzthiazolin)-6-sulfonsäure) verwendet und nach erfolgter Farbreaktion wurde die optische Dichte (OD) bei 405 nm gemessen.

### PCSK9 Protein-ELISA:

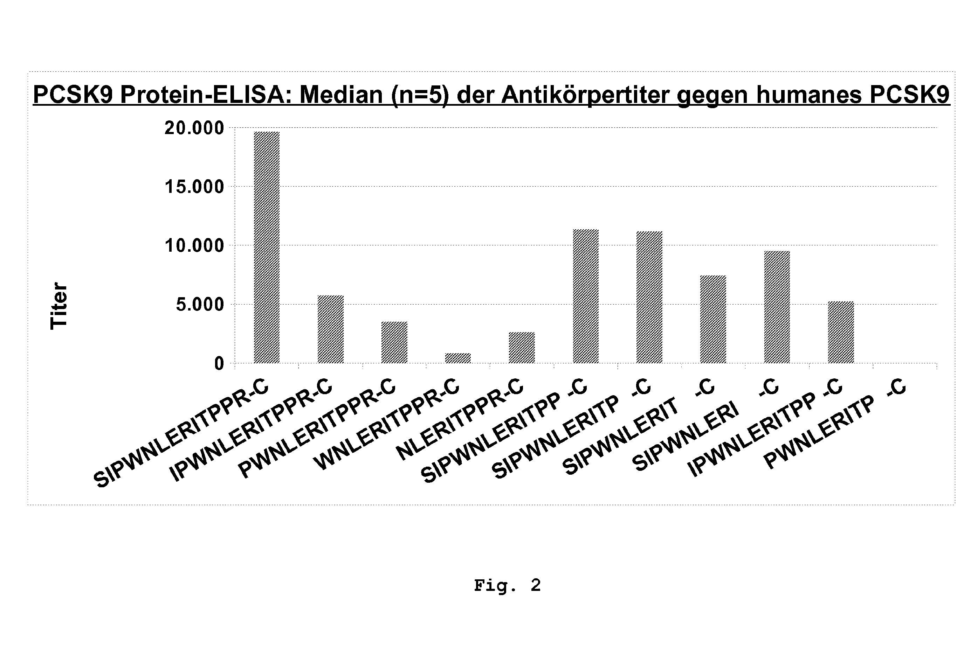

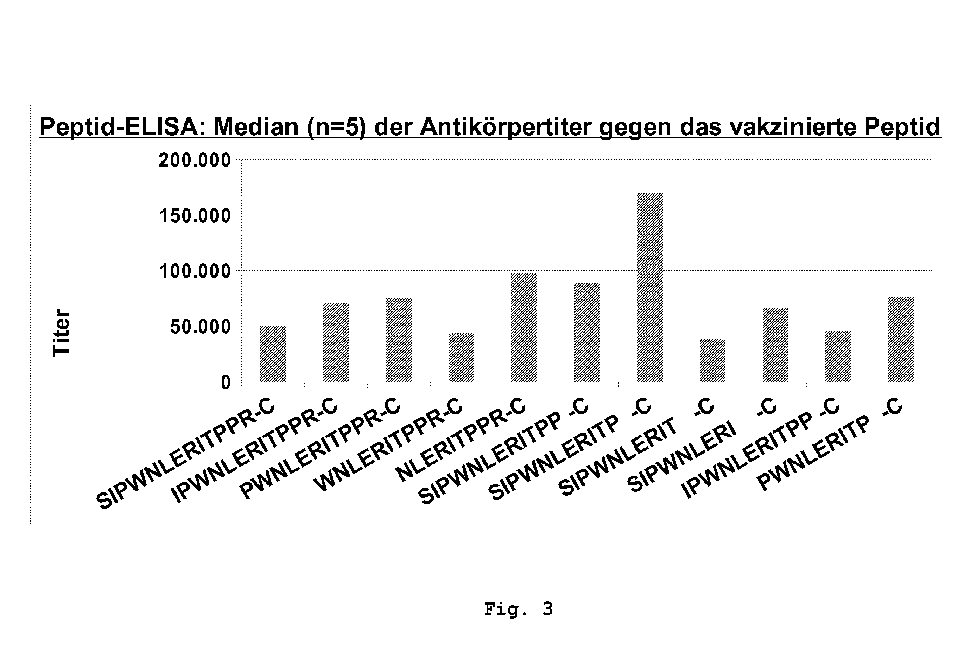

Die Durchführung des Protein-ELISAs erfolgte wie der Peptid-ELISA, nur dass anstelle des Peptid-BSA Konjugats rekombinantes humanes PCSK9 immobilisiert wurde (siehe Figuren 2 und 3 und folgende Tabelle).

| **SEQ ID Nr.** | **anti-PCSK9 Titer** | | | | | **Median** | |
|---|---|---|---|---|---|---|---|
| | *Maus 1* | *Maus 2* | *Maus 3* | *Maus 4* | *Maus 5* | **Protein-Titer** | **Peptid-Titer** |
| 2 | 19.629 | 15.340 | 19.984 | 2.827 | 38.175 | 19.629 | 50.531 |
| 3 | 3.653 | 7.607 | 5.707 | 2.978 | 10.556 | 5.707 | 71.149 |
| 4 | 3.490 | 10.241 | 300 | 3.687 | 1.008 | 3.490 | 75.701 |
| 5 | 500 | 0 | 2.554 | 795 | 3.492 | 795 | 44.409 |
| 6 | 5.562 | 1.455 | 2.577 | 2.236 | 18.858 | 2.577 | 98.149 |
| 7 | 36.853 | 9.407 | 16.987 | 11.310 | 3.980 | 11.310 | 88.883 |
| 8 | 12.427 | 6.700 | 11.124 | 10.561 | 32.751 | 11.124 | 169.855 |
| 9 | 7.777 | 5.776 | 41.508 | 7.419 | 2.228 | 7.419 | 38.970 |
| 10 | 4.767 | 5.303 | 24.630 | 15.053 | 9.502 | 9.502 | 66.643 |
| 11 | 19.701 | 5.224 | 5.975 | 2.497 | 1.170 | 5.224 | 46.047 |
| 12 | 0 | 0 | 0 | 0 | 0 | 0 | 7.6797 |

Sämtliche verabreichten Peptide sind in der Lage, die Bildung von Antikörpern gerichtet gegen dieselbigen zu induzieren. Die Verabreichung der Peptide mit SEQ ID Nr. 2, 7, 8, 9 und 10 führte zudem zu einer erhöhten Bildung von Antikörpern gerichtet gegen PCSK9.

### Beispiel 4: PCSK9-LDLR (Bindungs-)Assay

Ein Antikörper, der gegen das V5-Tag gerichtet ist (polyklonaler Kaninchen anti-V5 Antikörper, Novus Biologicals), wird in einer Konzentration von 1 µg/ml in PBS verdünnt und auf einer ELISA Platte immobilisiert. Nach dem Abblocken der nicht besetzten Stellen wurde die Platte mit rekombinantem humanen PCSK9, das C-Terminal ein V5-Tag gefolgt von einem 6xHis-Tag aufweist, inkubiert und dadurch PCSK9 "abgefangen". Anschließend folgte eine Prä-Inkubation des gebundenen PCSK9 mit Serum von vakzinierten Mäusen. Dafür wurde das Serum auf eine 15 % Konzentration in Bindungspuffer (20 mM Tris-HCl, pH 6.0, 100 mM NaCl, 1 mM CaCl₂, 0,1 % Tween20) verdünnt und jeweils 25 µl/Well zugegeben. Nach einer Inkubation von 30 min bei Raumtemperatur wurden jeweils 25 µl/Well eines rekombinanten humanen LDLR (Low Density Lipoprotein Receptor, R&D Systems), der auf 1,5 µg/ml in Bindungspuffer verdünnt wurde, dazugegeben und zusammen mit den Mausseren inkubiert. Nach einer Inkubation für 2 Stunden bei Raumtemperatur wurden alle nicht gebundenen Proteine weggewaschen. LDLR, der an das abgefangene PCSK9 gebunden ist, wurde mit einem anti-LDLR Antikörper (polyklonaler Huhn anti-LDLR Antikörper, Abcam), gefolgt von einem biotinylierten anti-Huhn IgG Antikörper und einem Streptavidin-HRP Konjugat detektiert. Als Substrat für die Peroxidase dient TMB (3,3',5,5'-Tetramethylbenzidin). Die Farbreaktion wurde mit einer Stop-Lösung beendet und die optische Dichte bei 450 nm gemessen.

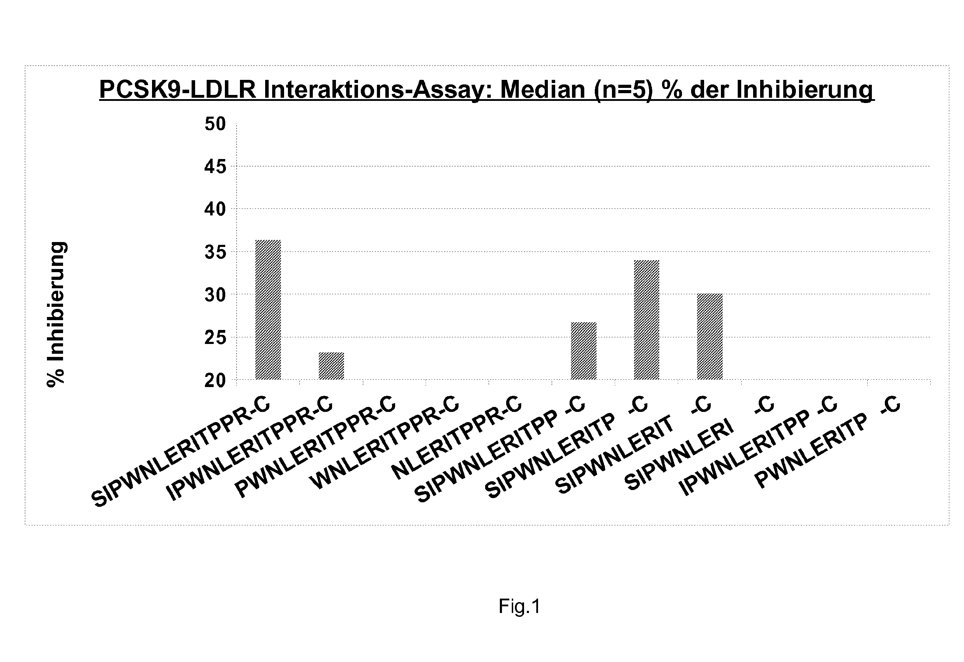

Die Berechnung der % Bindung bzw. Inhibierung erfolgte folgendermaßen. Seren von 5 Kontroll-Mäusen wurden in jedem Assay inkludiert. Der Durchschnitt dieser Werte wurde berechnet und als 100 % Bindung (entspricht 0 % Inhibierung) angenommen. Die Standardabweichung der ermittelten Bindung wurde ebenfalls berechnet und mit 2 multipliziert. Alle Werte, die unter der 2x Standardabweichung lagen, wurden als Inhibierung angenommen. Bei den gezeigten Ergebnissen beträgt diese 2x Standardabweichung ∼22 % (siehe folgende Tabelle und Fig. 1).

| **SEQ ID Nr.** | **% Inhibierung der PCSK9-LDLR Interaktion** | | | | | **Median** |
|---|---|---|---|---|---|---|
| | *Maus 1* | *Maus 2* | *Maus 3* | *Maus 4* | *Maus 5* | |
| 2 | 8 | **35** | **52** | **44** | **36** | **36** |
| 3 | 23 | 22 | 9 | **28** | **32** | 23 |
| 4 | 26 | 0 | 0 | 18 | 19 | 18 |
| 5 | 0 | 0 | 0 | 17 | 9 | 0 |
| 6 | 26 | 12 | 0 | 10 | **34** | 12 |
| 7 | 23 | **46** | **37** | 5 | **27** | **27** |
| 8 | **49** | **48** | **34** | 12 | **33** | **34** |
| 9 | 0 | **43** | **27** | **33** | **30** | **30** |
| 10 | 6 | 0 | 7 | **31** | 13 | 7 |
| 11 | 16 | 9 | 11 | 22 | 0 | 11 |
| 12 | 4 | 9 | 0 | 0 | 0 | 0 |

Die Ergebnisse zeigen, dass insbesondere die Peptide mit der SEQ ID Nr. 2, 7, 8 und 9 in der Lage sind, die Interaktion zwischen PCSK9 und LDL-Rezeptor zu inhibieren.

## Patentansprüche

1. Peptid, welches aus 10 bis 30 Aminosäureresten besteht und von der Aminosäuresequenz SIPWNLERITPPRYRADEYQPPDGGSLVEV (SEQ ID Nr. 1) abgeleitet ist, **dadurch gekennzeichnet, dass** das Peptid die Aminosäuresequenz SIPWNLERIT umfasst.

2. Peptid nach Anspruch 1, **dadurch gekennzeichnet, dass** das Peptid die Aminosäuresequenz SIPWNLERITPPR, SIPWNLERITPP, SIPWNLERITP oder SIPWNLERIT aufweist.

3. Peptid nach Anspruch 1 oder 2, **dadurch gekennzeichnet, dass** das Peptid am C-Terminus und/oder am N-Terminus einen Cystein-rest aufweist.

4. Peptid nach einem der Ansprüche 1 bis 3 zur Verwendung in einem Verfahren zur Behandlung und/oder Prävention von durch Atherosklerose verursachten Störungen, insbesondere kardiovaskulären Erkrankungen, Schlaganfällen oder peripheren vaskulären Erkrankungen.

5. Peptid nach Anspruch 4, **dadurch gekennzeichnet, dass** das Peptid einem Patienten in einer Menge von 0,1 ng bis 10 mg, vorzugsweise von 1 µg bis 500 µg, verabreicht wird.

6. Vakzin umfassend ein Peptid nach einem der Ansprüche 1 bis 3.

7. Vakzin nach Anspruch 6, **dadurch gekennzeichnet, dass** das Peptid an einen pharmazeutisch akzeptablen Träger, vorzugsweise an KLH (Keyhole Limpet Hämocyanin), gekoppelt ist.

8. Vakzin nach Anspruch 6 oder 7, **dadurch gekennzeichnet, dass** das Peptid zur intradermalen, subkutanen oder intramuskulären Verabreichung formuliert ist.

9. Vakzin nach einem der Ansprüche 6 bis 8, **dadurch gekennzeichnet, dass** das Peptid mit einem Adjuvans, vorzugsweise mit Aluminiumhydroxid, formuliert ist.

10. Vakzin nach einem der Ansprüche 6 bis 9, **dadurch gekennzeichnet, dass** das Peptid in einer Menge von 0,1 ng bis 10 mg, vorzugsweise 10 ng bis 1 mg, insbesondere 100 ng bis 100 µg, pro Dosis im Vakzin enthalten ist.

11. Vakzin nach einem der Ansprüche 6 bis 10 zur Verwendung in einem Verfahren zur Behandlung und/oder Prävention von durch Atherosklerose verursachten Störungen, insbesondere kardiovaskulären Erkrankungen, Schlaganfällen oder peripheren vaskulären Erkrankungen.
